# EUROPEAN PATENT SPECIFICATION

(11) **EP 0 619 720 B1**
(45) Date of publication and mention of the grant of the patent: **24.02.1999**
(21) Application number: 93902829.6
(22) Date of filing: 30.12.1992
(51) Int. Cl.: A61B 17/00, A61F 2/01

(54) **THROMBOSIS FILTER**
THROMBOSEFILTER
FILTRE DE THROMBOSE

(30) Priority: 30.12.1991 JP 113712/91 U
(43) Date of publication of application: 19.10.1994
(73) Proprietor: SCIMED LIFE SYSTEMS, INC., Maple Grove, MN 55369-7503 (US)
(72) Inventor: IRIE, Toshiyuki, Tokorozawa Saitama (JP); FRUI, Shigeru, Tokorozawa Saitama (JP)
(74) Representative: Baverstock, Michael George Douglas
(86) International application number: US9211311
(87) International publication number: WO9312723

(56) References cited:
- US-A- 4 425 908
- US-A- 4 832 055

## Description

### BACKGROUND OF THE INVENTION

The present invention relates to thrombosis filters. To be more specific, it is about removable thrombosis filters which can be securely fixed at a selected location in the vascular system for trapping blood clots, and removed when desired such as when no longer required.

In recent years, pulmonary embolism has become an increasingly common medical emergency. Pulmonary embolisms may be caused by venous thrombosis that in turn may be caused by blood flow retention, venous intimal damage or coagulation abnormalities. Emergency or prophylactic treatments for these conditions include anticoagulant therapy, thrombolytic therapy, thrombectomy and inferior vena cava blocking procedures.

Among these therapeutic options, when an inferior vena cava blocking procedure is selected, one option is to perform a laparotomy under general anesthesia during which the inferior vena cava is ligated, sutured and shortened, or clipped. Another option is to intravenously insert a thrombosis filter into the inferior vena cava under local anesthesia. A laparotomy however, retires general anesthesia and is susceptible to thrombosis formation due to the discontinuation of anticoagulant therapy prior to surgery. For these reasons, percutaneous filter insertion is now more widely employed since it retires only local anesthesia.

Blood is returned to the heart from the lower part of the human body through the inferior vena cava and from the upper part of the body through the superior vena cava. The neck vein, known as the jugular vein, leads to the superior vena cava which is a vein that enters the upper part of the heart. A percutaneously inserted catheter that extends through the jugular vein and the superior vena cava and then into the heart can be manipulated to exit the heart through the mouth of the inferior vena cava into the heart. The inferior vena cava, a vein that enters the lower part of the heart, can also be accessed through the patients femoral vein. Thus, the inferior vena cava can be reached through two endovenous routes, both of which are available percutaneously.

A currently used percutaneous filter is shown in Figure 9. The filter has anchors, 43, at the end of six struts, (31), with a head or central hub or fixation part, 5. One example of how this filter is used is to lead the filter within a capsule with the head of the filter introduced first if using the transfemoral vein approach or with the tail of it introduced first if using the transjugular vein approach. This type of filter possesses hooks at the end of the struts that impinge the inner wall of the blood vessel in order to ensure its firm fixation at the time of insertion. It is released from the capsule and expands within the inferior vena cava where it functions as thrombosis filter.

Filters of the type shown in Figure 9 perform the desired filtering function, requiring only local anesthesia for insertion. This has contributed to the high usage of the filter. A filter of the type shown in Figure 9 is disclosed in U.S. Patent No. 4,817,600. Approximately two to three weeks after insertion of this type of filter, neointima grows around the filter and its anchors to the inner wall of the blood vessel, which becomes an obstacle to the removal of the filter after its function has been fulfilled. Furthermore, this filter was not designed or intended to be removed.

US-A-4 832,055 describes a mechanically locking blood clot filter comprising wire portions forming a filter mesh and having leg portions each having a foot at the end for engaging an interior wall of a blood vessel to maintain the filter in position, the filter having at one end only a hook which is described as a "retractor" but whose function is, as explained in the specification, to retract a central core wire to a position where the filter is locked in its deployed position.

Since the need for a thrombosis filter, in many patients, is temporary, it may be desirable to remove the filter when it is no longer needed. In the prior art devices, such removal can be preformed surgically which exposes the patient to the usual risk and trauma associated with surgery. Thus there is a need for a thrombosis filter that can be removed without surgery.

The subject invention fulfills the need of a medical filter which can be non-surgically removed even after neointima has developed. As a result of this invention, a medical filter has been developed which includes insertion and removal accessories that enable the goal to be achieved. The filter of this invention includes a filtering portion that is permeable to the blood flow but which will filter out emboli and thrombus and will also hold the filter firmly in a selected location and permit removal through an endovenous route. The filtering and holding device of this medical filter includes two filter units that face each other and do not have hooks that impinge the inner wall of the blood vessel, in the same manner as the prior art filters, in order to anchor the device in the blood vessel.

The objective of this invention, therefore, is to provide thrombosis filters with a favorable filtering function which can be securely anchored at a desired location and be removed through an endovenous route even after the growth of neointima.

### SUMMARY OF THE INVENTION

According to the present invention there is provided a thrombosis filter adapted to be percutaneously implanted, in and removed from a blood vessel having an inner wall, through an endovenous route via a tube, said thrombosis filter comprising:
a filtering and holding device that extends along a longitudinal axis and is flexible in its radial dimension,
said filtering and holding device being dimensioned such that it can be compressed radially into a collapsed condition in which it can be received in said tube and will open out and expand into biased engagement with the inner wall of said blood vessel when it emerges from the tube,
   characterised in that said filtering and holding device includes coupling mechanisms at both ends, said coupling mechanisms accessible through endovenous routes to enable the filtering and holding device to be seized at both ends of the device, released from the inner wall of said blood vessel and withdrawn percutaneously from the blood vessel.

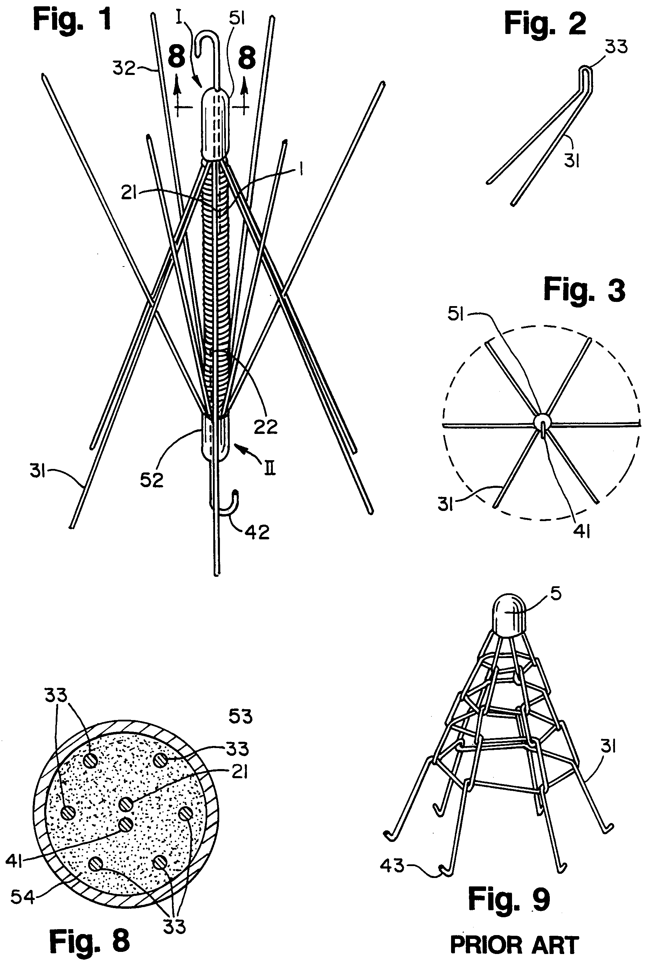

The above-mentioned objective is achieved by a specific thrombosis filter which is characterized by the following structure. The thrombosis filter of this invention incudes a first and a second filtering and holding unit each unit including a coupling mechanism in the form of a hook. Each unit has a plurality of resilient struts which radiate from a central hub and lies on the surface of a cone. The units are interconnected by a core shaft which consists of a compression spring and a pair of core wires. The term compression spring means a spring that will draw the filtering and holding units toward each other to thereby minimize the overall longitudinal length of the filter. Inside the core shaft, there are core wires that are anchored in the units and have free ends which extend from their anchored ends toward the other unit. The struts are resilient and their free ends bear against the inner wall of the vein. However, the free ends do not include anchoring hooks, of the type disclosed in the prior art, that impinge the wall of the vein as does the above discussed prior art filter. The anchoring of the filter, of this invention, is accomplished by orientating the two filter units in opposite directions. The force from the compression springs pulls the hubs from the two units toward each other, which tends to open the struts of both units thus forcing their free ends into the wall of the vein and holding the filter in its selected location. More specifically, the core shaft of the upper unit (as seen in Figure 1) consists of multiple struts which spread radially in an expansive direction while the core shaft of the lower unit consists of multiple struts which spread radially. The struts from the first and second units cross each other between their hubs and free ends, which when considering the conical surfaces that the struts lie upon, defines a V-shaped circular filtering grove. This V-shaped circular filtering groove is permeable to the flow of blood and performs an improved filtering function.

Although materials of the present thrombosis filters are not particularly restricted, it is desirable to use a flexible material that will return to its original shape after being deformed such as identity elastic alloy wire, high elastic alloy wire such as stainless steel, tungsten, platinum, piano wire, shape memory alloy wire, super elastic metal wire and chromium alloy. It is important that the coupling mechanisms in the form of hooks and the cord be constructed of material such a tungsten, platinum or gold that can be seen on a fluoroscope, to aid in the process of securing the coupling mechanisms.

Multiple number of struts or thrombosis filtering wires used for the present invention are divided into two units and are placed and anchored around their corresponding central hub at regular intervals to the extent possible. The struts of each unit lie on the surface of a cone with an anchoring part at the apex. The anchoring part is connected to and a part of the central hub.

Inside the compression spring, which constitutes the core shaft, a core wire extends from each of the filter units and function to reinforce the compression spring.

The angle between the radially spread struts and the core shaft can be optional, although it is preferable to maintain this angle within the range of 70 to 80 degrees.

The thickness of the wire constituting a strut is preferably 0.05 to 0.2 mm in diameter.

The ideal outside diameter of the compression spring is 0.5 to 2 mm in diameter, but there is no particular restriction to it. This diameter can of course be determined by the particular use intended for the filter.

As for the size of the thrombosis filters, there is no particular restriction. The size can be changed at one's discretion depending on the site of its application.

The present invention enables thrombosis filtering through multiple struts which spread radially. As a result the filtering and holding device is flexible in its radial dimension and can be compressed or collapsed radially for insertion, travel and removal. Since the struts do not have hooks for impinging the inner wall of the blood vessel, even when the struts are covered by neointima, they can be easily removed by pulling them towards the head or central hub. The compression spring, which serves as the core shaft, provides a core shaft that is flexible and facilitates maneuvering the filter units into the recovery tubes for removal. The compression spring allows relative movement of the units along the filter's longitudinal axis.

The core wires within the compression spring function to maintain the core shaft along a central axis extending between the two filter units as well as to achieve a desired flexibility of the compression spring.

The coupling mechanism, in the form of a hook element provided at the head of each filter unit, functions to connect the unit to mechanism that can be manipulated through an endovenous route at the time of removal.

As for the structure of the filter units facing each other, this arrangement serves to stabilize and anchor the filter inside the blood vessel and to prevent unfavorable phenomenon such as the displacement of the filter inside the vessels.

### BRIEF DESCRIPTION OF THE DRAWINGS

Figure 1 is a perspective view showing the thrombosis filter of this invention.
Figure 2 is a perspective view showing a strut of the thrombosis filter of this invention.
Figure 3 is an top view of filter unit I of the thrombosis filter of this invention.
Figure 4 is a cross-sectional view illustrating a step in the method for removing the thrombosis filter of this invention from the patient.
Figure 5 is a cross-sectional view illustrating a step in the method for removing the thrombosis filter of this invention from the patient.
Figure 6 is a cross-sectional view illustrating a step in the method for removing the thrombosis filter of this invention from the patient.
Figure 7 is a cross-sectional view illustrating a step in the method for removing the thrombosis filter of this invention from the patient.
Figure 8 is a cross-section view of the head portion of the thrombosis filter of this invention taken along line 8-8 of Figure 1.
Figure 9 is a perspective view of a conventional prior art thrombosis filter.

### DETAILED DESCRIPTION OF THE PREFERRED EMBODIMENT

The following is a discussion of how the present invention can be applied to a particular embodiment. This, however, does not mean that the application of the present invention is limited only to the following use.

Figure 1 is a perspective view, of the filtering and holding device of the preferred embodiment of the present invention. The filter is fabricated mainly from stainless steel wires. The wire is 0.05 to 0.2 mm in diameter. The filter consists of twin units, I and II, that are spaced from each other along the filtering and holding device's longitudinal axis and which face each other and are connected by the compression spring 1.

The filter unit I includes the following structure. The upper end of core wire 21, as viewed in Figure 1, extends downwardly from hook 41. Struts 31 also extend downwardly from hook 41 and spread radially in an expansive direction, centering around the core wire 21. The upper ends of struts 31 are fixed in the central hub or fixation part 51.

Filter unit II has the following structure. As viewed in Figure 1, the core wire 22 and struts 32 extends upwardly from hook 42. Struts 32 spread radially at an angle to the filtering and holding device's longitudinal axis in an expansive direction, centering around the core wire 22. The struts 32 are anchored at, their lower end in central hub or fixation part 52.

The free ends of the struts for both units I and II are thus spring biased outwardly and can be forced inwardly to decrease the diameter of the filtering and holding device when it is located in the insertion and removal tubes.

As shall be discussed in greater detail, a welding or binder material is normally used for brazing the ends of the hooks 41 and 42 the core wires 21 and 22 the struts 31 and 32 and the ends of spring 1 to the central hub or fixation parts 51 and 52.

The filter units I and II are spaced from each other along the filtering and holding device's longitudinal axis such that struts 31 and 32 crossed each other at about their mid points. Hooks 41 and 42 are constructed of tungsten or stainless steel wires that have been bent into the shape shown in Figure 1.

First ends of core wires 21 and 22 are connected to the central hub or fixation parts 51 and 52 along with the straight ends of hooks 41 and 42. The core wires 21 and 22 extend through the lumen formed by compression spring 1 and function as a core shaft that enhances the flexibility of compression spring 1. The other ends of core wires 21 and 22 are free.

Figure 2 is a diagonal view of a strut 31 that includes a head 33. Strut 32 is identical to strut 33 and thus is not illustrated in an isolated view. The heads 33 of struts 31 and 32 become a part of the core shaft by being anchored in central hub or fixation parts 51 and 52.

Figure 3 is a top view of filter unit I. As can be seen in this view, struts 31 emanate from the central core shaft and spread outwardly from this center in equal amounts into the surrounding area. Although six struts 31 in each filter unit have been found to be optimum, this number can be varied within the range of three to ten.

Figure 8 includes two enlarged cross-sectional views taken through the central hub or fixation part 51. A tube 53 is used to form the central hub or fixation part 51 of the thrombosis filter. The straight end of hook 41, first end of core wire 21 and the heads 33 of struts 31 are all inserted into the tube 53. The ends of spring 1 could also be inserted into the tube 53 and bonded to the tube 53 or the ends could be fixed to the tube by welding or adhesive. A bonding material 54 is then introduced, while in a pliable stage, into tube 53 such that the bonding material 54 fills all the voids between the ends of the hook 41, core wire 21 and heads 33 within tube 53. When the pliable material 54 hardens the ends of the hook 41, core wire 21 and the heads 33 are all fixed in the central hub or fixation part 51. The tube 53 could remain as a permanent part of central hub or fixation part 51 or it could be removed.

It should be noted that the compression spring 1, hooks 41 and 42, core wires 21 and 22 and the struts 31 and 32 can all be fabricated from the same material or each of these parts could be fabricated from different material that will provide the desired characteristics for the particular part.

The following is an explanation of how this embodiment of the thrombosis filter is used. The thrombosis filter is dimensioned such that it can be stored in the distal end of a thin tube (2 to 3 mm in diameter). This insertion tube is percutaneously inserted into the patient and follows an endovenous route into the patient's inferior vena cava. This procedure is performed under local anesthesia. When the distal end of this tube reaches the target site, the filtering and holding device stored inside the tube is caused to exit the distal end of the tube where it become implanted in the patient's inferior vena cava. A pusher rod is extended through the insertion tube and is maneuverable from the proximate end of the insertion tube for expelling the filtering and holding device out the distal end of the insertion tube. It should be noted that the insertion procedure can be monitored on a fluoroscope.

The filtering and holding device after being released from the tube into the patient's inferior vena cava, through the above described procedure, is in the form as shown in figure 1. Within several week after the filtering and holding device has been implanted in the inferior vena cava, the struts 31 and 32, which are in contact with the inferior vena cava become covered by neointima.

When it becomes necessary or desirable to remove the filtering and holding device, the following procedure is followed. The term "medical filter" as used to describe and claim this embodiment includes in addition to the filtering and holding device the above mentioned insertion tube and the recovery hardware necessary to remove the filter. A recovery device is provided that can be inserted percutaneously and threaded through the endovenous route to the filtering and holding device. The recovery device includes a first tube 64, a second tube or sheath 61, a third tube 63 and a recovery tube or sheath 62.

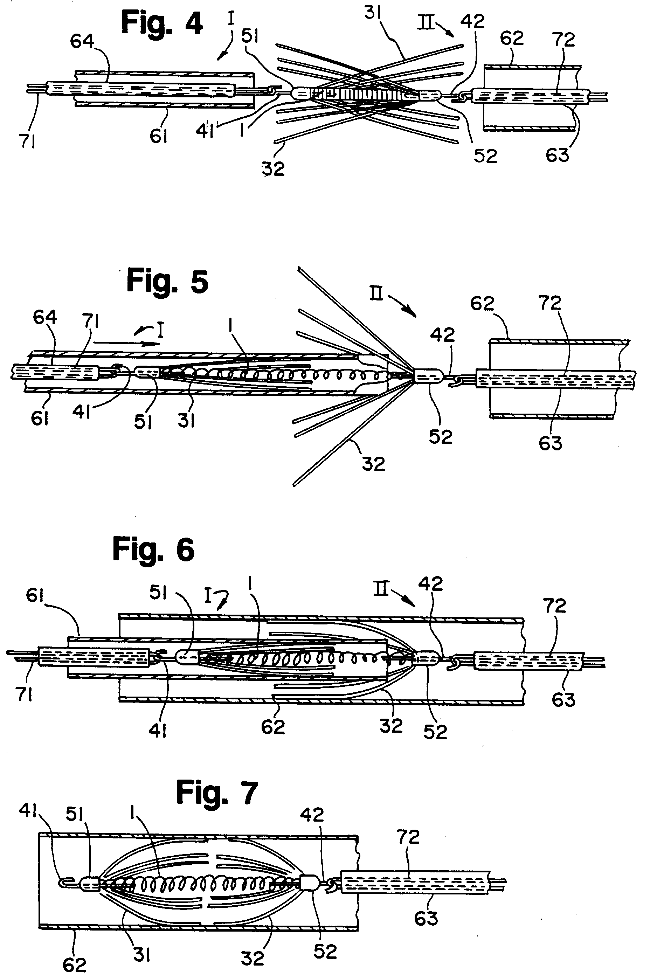

Referring to figure 4, a doubled over cord 71 inserted through the proximal end of the first tube 64 such that the cord emerges at the distal end of the tube 64 and functions as a coupling mechanism in the form of a loop. The tube 64 then is passed through the second tube or sheath 61 which has been percutaneously inserted into the patient via the femoral vein such that its distal end is in the inferior vena cava of the patient adjacent the filtering and holding device. The tube 64 is advanced, through tube or sheath 61, to the filtering and holding device. The tube 64, that has the loop formed in cord 71 emerging from its distal end, is manipulated such that the coupling mechanism in the form of a loop is grasp by the complementary coupling mechanism in the form of a hook 41.

In the same manner, a doubled over cord 72 is passed through third tube 63 such that a loop that functions as a coupling mechanism emerges from the distal end of the tube. Third tube 63 is threaded through a recovery tube or sheath 62 which was percutaneously inserted through the right internal jugular vein such that its distal end is in the patients inferior vena cava. The third tube 63 is manipulated such that the coupling mechanism in the form of a loop is grasped by the complementary coupling mechanism in the form of a hook 42. After cord 64 is connected to hook 41 and cord 72 is connected to hook 42 the cords 64 and 72 are simultaneously pulled in opposite directions, causing units I and II to move away from each other. The compression spring 1 expands to permit this relative movement of units I and II. A tension is maintained on the cords 64 and 72 to insure that the connection between the cords 64 and 72 and the hooks 41 and 42 is maintained.

Cords 64 and 72 are made of or include radiopaque material such as stainless steel so the at the coupling of the loops with hooks 41 and 42 can be monitored of a scope.

The relative movement of units I and II has resulted in a corresponding movement of the struts 31 and 32 relative to the vessel wall and the filters have been freed from the neointima.

The next step in the process of removing the filtering and holding device from the patient is illustrated in Figure 5. The cord 71 is pulled in its proximal direction to thus pull the filter unit 1 into tube or sheath 61 for temporary storage. Then tube 64 is advanced further through tube or sheath 61 to the point where its distal end is adjacent the ends of struts 32 that are secured in the fixation member 51 of unit II.

As can be best seen in Figure 6, the next step in the removal procedure is to advance tube or sheath 62, in the direction toward its distal end, such that unit II and the distal end of tube or sheath 61 in which is stored unit I, are received within tube or sheath 62.

The entire filtering and holding device is now stored in recovery tube or sheath 62. By pulling one strand of cord 71, from its proximal end, cord 71 is released from hook 41 and can be removed from the patient. The second tube or sheath 61 can then be removed from the patient, and as shown in Figure 7, and the entire filtering and holding device is now stored in recovery tube or sheath 62.

The final step in the process for removing the filtering and holding device from its resting place in the patient's inferior vena cava is to pull out the recovery tube or sheath 62.

When the filters used for the present invention are employed in vessels, in order to prevent the adhesion of thrombosis it is preferable to coat the filters with antithrombotic agents (such as heparin, urokinase and antithrombotic material including hydroxy methacrylate-styrene copolymer).

As a result of the thrombosis filters units I and II being connected by a compression spring with the struts 31 and 32 extending in opposite directions, fixation of the filtering and holding device at a selected location is accomplished upon release of the filter from the tube. In addition, the arrangement of the struts 31 and 32 extending in opposite directions and crossing provides a very effective thrombus filter. Moreover, the thrombus filter of this invention can be removed when the patient no longer has a need for it.

## Claims

1. A thrombosis filter adapted to be percutaneously implanted, in and removed from a blood vessel having an inner wall, through an endovenous route via a tube, said thrombosis filter comprising:
a filtering and holding device that extends along a longitudinal axis and is flexible in its radial dimension,
said filtering and holding device being dimensioned such that it can be compressed radially into a collapsed condition in which it can be received in said tube and will open out and expand into biased engagement with the inner wall of said blood vessel when it emerges from the tube,
characterised in that said filtering and holding device includes coupling mechanisms (41, 42) at both ends, said coupling mechanisms accessible through endovenous routes to enable the filtering and holding device to be seized at both ends of the device, released from the inner wall of said blood vessel and withdrawn percutaneously from the blood vessel.

2. A thrombosis filter as claimed in claim 1 further characterised in that said thrombosis filter includes first and second portions (I, II) displaced longitudinally and joined by a resilient member (1) that enables said first and second portions to move longitudinally relative to each other to facilitate releasing the first and second portions from the inner wall during removal.

3. A thrombosis filter as claimed in claim 1 or claim 2 further characterised in that said thrombosis filter includes a recovery device (63, 64) that can be inserted percutaneously and threaded through an endovenous route such that its distal end is located at said filtering and holding device, and
coupling mechanism (71, 72) carried by said recovery device at its distal end, said coupling mechanism carried by said recovery device being complementary to said coupling mechanism (41, 42) of said filtering and holding device such that said filtering and holding device can be seized by said recovery device.

4. A thrombosis filter as claimed in claims 1 or 2 further characterised in that said coupling mechanisms (41, 42, 71, 72) on the filtering and holding device are made of radiopaque material to assist in the process of snaring the filtering and holding device.

5. A thrombosis filter as claimed in claim 3 further characterised in that said thrombosis filter includes a recovery tube (61, 62) having an open distal end dimensioned to receive the collapsed filtering and holding device,
said recovery tube adapted to be inserted percutaneously into the patient and threaded over an endovenous route such that its open distal end is adjacent the filtering and holding device, said recovery device functioning to move the filtering and holding device from its location within the blood vessel to a location within the distal end of said recovery tube.

6. A thrombosis filter as claimed in any one of the preceding claims to be placed in the blood vessel of a patient for trapping clots, the filter being adapted to be inserted percutaneously through an insertion tube or tubes and expelled from the insertion tube or tubes at a selected location in the blood stream, where it implants itself in the inner wall of the blood vessel so as to prevent migration of the filter within the blood vessel,
said filter includes a first and second unit (I, II) that are spaced from each other along the filter's longitudinal axis,
characterised in that each of said units includes a plurality of spurs (31, 32) anchored such that their free ends radiate outwardly at an acute angle to said longitudinal axis in the direction toward the other of said units, said plurality of spurs open out from a collapsed condition when the filter is released from the insertion tube or tubes into the blood vessel at the desired position causing the free ends to engage the inner wall of said blood vessel,
one or more of said spurs functioning to filter out clots that are flowing through the blood vessel,
one or more of said spurs having free ends that are forced into the side wall of the blood vessel as a result of a force attempting to move the filter axially of the blood vessel, one or more of said free ends functions as a holding device to anchor the filter at the desired position within the blood vessel,
said insertion tube or tubes including a pusher that is maneuverable from the proximal end of the insertion tube or tubes for expelling the filter out the distal end of the insertion tube or tubes.

7. A thrombosis filter as claimed in claim 6 wherein
said first and second units (I, II) are interconnected by a resilient member (1) extending along said longitudinal axis characterised in that said resilient member exerts a force on said units causing the free ends of the spurs 31, 32 to grip the inner wall of the blood vessel and hold the filter in its desired position.

8. A thrombosis filter as described in claim 7 further characterised in that each unit I, II of said filter includes a core wire (21, 22), one end of each core wire is anchored to one of said units and extends from its anchor along said resilient member toward the other unit, said core wires terminating in free ends short of said other unit.

9. A method of constructing a thrombosis filter having coupling mechanisms at both ends characterised by steps of:
(a) providing a tube having open ends;
(b) inserting an end of a coupling mechanism into one of the open ends of said tube;
(c) inserting the ends of a plurality of outwardly diverging spurs into the other open end of said tube;
(d) inserting an end of a core wire into said other open end of said tube;
(e) filling all remaining space in the tube between its open ends with a bonding material that will harden to secure the coupling mechanism, plurality of spurs and core wire in the tube.

10. A method as claimed in claim 9 including the additional steps of:
(f) providing a second tube having open ends;
(g) inserting an end of a coupling mechanism in one of the open ends of said second tube;
(h) inserting the ends of a plurality of outwardly diverging spurs into the other open end of said second tube;
(i) inserting an end of a core wire into said other open end of said second tube;
(j) connecting one end of a resilient member to said other end of said tube;
(k) connect the other end of said resilient member to said other end of said second tube; and
(l) filling all remaining space in said second tube between its open ends with a bonding material that will harden to secure the coupling mechanism, plurality of spurs and core wire in the tube.

11. A method as claimed in claim 9 in which the diverging spurs have free ends extending from said tube that lie along the surface of a cone that has its apex within said tube.

12. A method as claimed in claim 10 in which the diverging spurs that have been bonded into said tube and said second tube have free ends extending from said tube and said second tube that lie along the surfaces of cones that have their apices within said tube and said second tube.

13. A method as claimed in claim 12 in which the diverging spurs extending from said tube cross the diverging spurs extending from said second tube.

## Patentansprüche

1. Thrombosefilter für die perkutane Implantierung in und Entfernung aus einem Blutgefäß mit einer Innenwand auf einem endovenösen Weg über ein Röhrchen, wobei der Thrombosefilter umfaßt:
eine Filter- und Haltevorrichtung, die sich längs einer Längsachse erstreckt und in ihrer radialen Abmessung flexibel ist,
wobei die Filter- und Haltevorrichtung derart bemessen ist, daß sie radial in einen zusammengelegten Zustand zusammengedrückt werden kann, in welchem sie in dem Röhrchen aufgenommen werden kann und sich in einen vorgespannten Eingriff mit der Innenwand des Blutgefäßes aufweitet und ausdehnt, wenn sie aus dem Röhrchen austritt,
**dadurch gekennzeichnet,** daß die Filter- und Haltevorrichtung Kupplungsmechanismen (41, 42) an beiden Enden umfaßt, wobei die Kupplungsmechanismen auf endovenösen Wegen zugänglich sind, um der Filter- und Haltevorrichtung zu ermöglichen, an beiden Enden der Vorrichtung erfaßt, von der Innenwand des Blutgefäßes gelöst und perkutan aus dem Blutgefäß herausgezogen zu werden.

2. Thrombosefilter nach Anspruch 1, ferner **dadurch gekennzeichnet,** daß der Thrombosefilter einen ersten und einen zweiten Abschnitt (I, II) umfaßt, die in Längsrichtung versetzt und durch ein elastisches Element (1) verbunden sind, das dem ersten und dem zweiten Abschnitt ermöglicht, sich in Längsrichtung relativ zueinander zu bewegen, um während der Entnahme das Lösen des ersten und des zweiten Abschnitts von der Innenwand zu erleichtern.

3. Thrombosefilter nach Anspruch 1 oder 2, ferner **dadurch gekennzeichnet,** daß der Thrombosefilter umfaßt:
eine Bergevorrichtung (63, 64), die perkutan eingeführt und derart auf einem endovenösen Weg eingefädelt werden kann, daß ihr distales Ende an der Filter- und Haltevorrichtung angeordnet ist, und
einen Kupplungsmechanismus (71, 72), der von der Bergevorrichtung an ihrem distalen Ende getragen ist, wobei der von der Bergevorrichtung getragene Kupplungsmechanismus derart zu dem Kupplungsmechanismus (41, 42) der Filter- und Haltevorrichtung komplementär ist, daß die Filter- und Haltevorrichtung durch die Bergevorrichtung erfaßt werden kann.

4. Thrombosefilter nach einem der Ansprüche 1 oder 2, ferner **dadurch gekennzeichnet,** daß die Kupplungsmechanismen (41, 42, 71, 72) an der Filter- und Haltevorrichtung aus einem radiopaken Material bestehen, um den Fangprozeß der Filter- und Haltevorrichtung zu unterstützen.

5. Thrombosefilter nach Anspruch 3, ferner **dadurch gekennzeichnet,** daß der Thrombosefilter ein Bergeröhrchen (61, 62) umfaßt, das ein offenes distales Ende hat, das zur Aufnahme der zusammengelegten Filter- und Haltevorrichtung dimensioniert ist,
wobei das Bergeröhrchen dafür eingerichtet ist, perkutan in den Patienten eingeführt und auf einem endovenösen Weg so eingefädelt zu werden, daß sein offenes distales Ende benachbart der Filter- und Haltevorrichtung ist, wobei die Bergevorrichtung funktioniert, um die Filter- und Haltevorrichtung von ihrem Ort innerhalb des Blutgefäßes zu einem Ort innerhalb des distalen Endes des Bergeröhrchens zu bewegen.

6. Thrombosefilter nach einem der vorangehenden Ansprüche zur Anordnung in dem Blutgefäß eines Patientens zum Einfangen von Gerinnseln, wobei der Filter dafür eingerichtet ist, perkutan durch ein oder mehrere Einführungsröhrchen eingeführt und aus dem oder den Einführungsröhrchen an einem ausgewählten Ort in den Blutstrom ausgestoßen zu werden, wo er sich selbst in die Innenwand des Blutgefäßes implantiert, um die Bewegung des Filters innerhalb des Blutgefäßes zu verhindern,
wobei der Filter eine erste und eine zweite Einheit (I, II) umfaßt, die längs der Längsachse des Filters voneinander beabstandet sind,
**dadurch gekennzeichnet,** daß jede der Einheiten eine Mehrzahl von Streben (31, 32) umfaßt, die derart verankert sind, daß ihre freien Enden sich in einem spitzen Winkel zur Längsachse in der Richtung auf die andere der Einheiten zu radial ausbreitet, wobei sich die Mehrzahl von Streben aus einem zusammengelegten Zustand aufweitet, wenn der Filter von dem oder den Einführungsröhrchen an der gewünschten Stelle in das Blutgefäß freigegeben ist, was das Eingreifen der freien Enden an der Innenwand des Blutgefäßes bewirkt,
wobei eine oder mehrere der Streben zum Ausfiltern von Gerinnsel funktionieren, die durch das Blutgefäß fließen,
wobei eine oder mehrere der Streben freie Enden haben, die infolge einer Kraft, die versucht, den Filter axial zum Blutgefäß zu bewegen, in die Seitenwand des Blutgefäßes gedrückt werden und wobei eines oder mehrere der freien Enden als eine Haltevorrichtung wirken, um den Filter an der gewünschten Stelle innerhalb des Blutgefäßes zu verankern,
wobei das oder die Einführungsröhrchen einen Ausstoßer umfassen, der von dem proximalen Ende des oder der Einführungsröhrchen zum Ausstoßen des Filters aus dem distalen Ende des oder der Einführungsröhrchen manövrierbar ist.

7. Thrombosefilter nach Anspruch 6, wobei die erste und die zweite Einheit (I, II) durch ein elastisches Element (1) miteinander verbunden sind, das sich längs der Längsachse erstreckt, **dadurch gekennzeichnet,** daß das elastische Element eine Kraft auf die Einheiten ausübt, die bewirkt, daß die freien Enden der Streben (31, 32) in die Innenwand des Blutgefäßes eingreifen und den Filter in seiner gewünschten Stellung halten.

8. Thrombosefilter nach Anspruch 7, ferner **dadurch gekennzeichnet,** daß jede Einheit (I, II) des Filters einen Kerndraht (21, 22) umfaßt, wobei ein Ende jedes Kerndrahts an einer der Einheiten verankert ist und sich von seiner Verankerung aus längs des elastischen Elements auf die andere Einheit zu erstreckt und wobei die Kerndrähte in freien Enden kurz vor der anderen Einheit enden.

9. Verfahren zur Konstruktion eines Thrombosefilters, der einen Kupplungsmechanismus an beiden Enden aufweist, **gekennzeichnet durch** die Schritte:
(a) Bereitstellen eines Röhrchens, das offene Enden hat;
(b) Einsetzen eines Endes eines Kupplungsmechanismus in eines der offenen Enden des Röhrchens;
(c) Einsetzen der Enden einer Mehrzahl von nach außen divergierenden Streben in das andere offene Ende des Röhrchens;
(d) Einsetzen eines Endes eines Kerndrahts in das andere offene Ende des Röhrchens;
(e) Füllen des gesamten verbleibenden Raums in dem Röhrchen zwischen seinen offenen Enden mit einem Bindemittel, das aushärtet, um den Kupplungsmechanismus, die Mehrzahl von Streben und den Kerndraht in dem Röhrchen festzulegen.

10. Verfahren nach Anspruch 9, umfassend die zusätzlichen Schritte:
(f) Bereitstellen eines zweiten Röhrchens, das offene Enden hat;
(g) Einsetzen eines Endes eines Kupplungsmechanismus in eines der offenen Enden des zweiten Röhrchens;
(h) Einsetzen der Enden einer Mehrzahl von nach außen divergierenden Streben in das andere offene Ende des zweiten Röhrchens;
(i) Einsetzen eines Endes eines Kerndrahts in das andere offene Ende des zweiten Röhrchens;
(j) Verbinden eines Endes eines elastischen Elements mit dem anderen Ende des Röhrchens;
(k) Verbinden des anderen Endes des elastischen Elements mit dem anderen Ende des zweiten Röhrchens; und
(l) Füllen des gesamten verbleibenden Raums in dem zweiten Röhrchen zwischen seinen offenen Enden mit einem Bindemittel, das aushärtet, um den Kupplungsmechanismus, die Mehrzahl von Streben und den Kerndraht in dem Röhrchen festzulegen.

11. Verfahren nach Anspruch 9, bei dem die divergierenden Streben freie Enden haben, die sich von dem Röhrchen aus längs der Oberfläche eines Konus liegend, der seinen Scheitel innerhalb des Röhrchens hat, erstrecken.

12. Verfahren nach Anspruch 10, bei dem die in dem Röhrchen und dem zweiten Röhrchen gebunden divergierenden Streben freie Enden haben, die sich von dem Röhrchen und dem zweiten Röhrchen aus längs der Flächen von Konen liegend, die ihre Scheitel innerhalb des Röhrchens und des zweiten Röhrchens haben, erstrecken.

13. Verfahren nach Anspruch 12, bei dem sich die von dem Röhrchen aus erstreckenden divergierenden Streben mit den von dem zweiten Röhrchen aus erstreckenden divergierenden Streben kreuzen.

## Revendications

1. Filtre de thrombose conçu pour implantation de façon percutanée dans un vaisseau sanguin, comportant une paroi interne, et retrait de celui-ci, par une voie intraveineuse par l'intermédiaire d'un tuyau, ledit filtre de thrombose comprenant :
un dispositif de filtrage et de maintien qui s'étend le long d'un axe longitudinal et qui est flexible dans sa dimension radiale,
ledit dispositif de filtrage et de maintien étant dimensionné de façon qu'il puisse être comprimé radialement dans un état replié dans lequel il peut être reçu dans ledit tuyau et s'évaser et se déployer en coopération rappelée avec la paroi interne dudit vaisseau sanguin lorsqu'il émerge du tuyau,
caractérisé en ce que ledit dispositif de filtrage et de maintien comprend des mécanismes (41, 42) de couplage aux deux extrémités, lesdits mécanismes de couplage étant accessibles par des voies intraveineuses pour permettre de saisir le dispositif de filtrage et de maintien aux deux extrémités du dispositif, de le libérer de la paroi interne dudit vaisseau sanguin et de le retirer de façon percutanée du vaisseau sanguin.

2. Filtre de thrombose selon la revendication 1, caractérisé en outre en ce que ledit filtre de thrombose comprend des première et seconde parties (I, II) décalées longitudinalement et reliées par un élément élastique (1) qui permet de déplacer lesdites première et seconde parties longitudinalement l'une par rapport à l'autre pour faciliter la libération des première et seconde parties de la paroi interne pendant le retrait.

3. Filtre de thrombose selon la revendication 1 ou la revendication 2, caractérisé en ce que ledit filtre de thrombose comprend un dispositif (63, 64) de récupération que l'on peut introduire de façon percutanée et conduire par une voie intraveineuse de façon que son extrémité distale se trouve au niveau dudit dispositif de filtrage et de maintien, et
un mécanisme (71, 72) de couplage porté par ledit dispositif de récupération à son extrémité distale, ledit mécanisme de couplage porté par ledit dispositif de récupération étant complémentaire audit mécanisme (41, 42) de couplage dudit dispositif de filtrage et de maintien de façon que ledit dispositif de filtrage et de maintien puisse être saisi par ledit dispositif de récupération.

4. Filtre de thrombose selon les revendications 1 ou 2, caractérisé en outre en ce que lesdits mécanismes (41, 42, 71, 72) de couplage situés sur le dispositif de filtrage et de maintien sont faits de matière radio-opaque pour favoriser le traitement de piégeage du dispositif de filtrage et de maintien.

5. Filtre de thrombose selon la revendication 3, caractérisé en outre en ce que ledit filtre de thrombose comprend un tuyau (61, 62) de récupération comportant une extrémité distale ouverte dimensionnée pour recevoir le filtre replié et le dispositif de maintien,
ledit tuyau de récupération étant apte à être introduit de façon percutanée dans le patient et conduit par voie intraveineuse de façon que son extrémité distale ouverte soit adjacente au dispositif de filtrage et de maintien, ledit dispositif de récupération fonctionnant pour déplacer le dispositif de filtrage et de maintien de son emplacement à l'intérieur du vaisseau sanguin à un emplacement à l'intérieur de l'extrémité distale dudit tuyau de récupération.

6. Filtre de thrombose selon l'une quelconque des revendications précédentes à placer dans un vaisseau sanguin d'un patient pour prendre au piège des caillots, le filtre étant adapté pour être introduit de façon percutanée dans un tuyau ou des tuyaux d'insertion et être sorti du tuyau ou des tuyaux d'insertion à un emplacement choisi dans le flux sanguin, là où il s'implante lui-même dans la paroi interne du vaisseau sanguin de façon à empêcher une migration du filtre à l'intérieur du vaisseau sanguin,
ledit filtre comprend un premier et un second module (I, II) qui sont espacés l'un de l'autre le long de l'axe longitudinal du filtre,
caractérisé en ce que chacun desdits modules comprend une pluralité d'aiguillons (31, 32) ancrés de façon que leurs extrémités libres rayonnent vers l'extérieur à un angle aigu par rapport audit axe longitudinal dans la direction vers l'autre desdits modules, ladite pluralité d'aiguillons s'évasant d'un état replié lorsque le filtre est libéré du tuyau ou des tuyaux d'insertion dans le vaisseau sanguin à la position souhaitée, en provoquant une coopération des extrémités libres avec la paroi interne dudit vaisseau sanguin,
un, ou plusieurs, desdits aiguillons fonctionnant pour filtrer les caillots qui circulent dans le vaisseau sanguin,
un, ou plusieurs, desdits aiguillons comportant des extrémités libres qui sont poussées dans la paroi latérale du vaisseau sanguin comme résultat d'une force tentant de déplacer le filtre axialement au vaisseau sanguin, une, ou plusieurs, desdites extrémités libres fonctionnent comme dispositif de maintien pour ancrer le filtre à la position souhaitée à l'intérieur du vaisseau sanguin,
ledit tuyau, ou lesdits tuyaux, d'insertion comprenant un poussoir qui peut être manoeuvré de l'extrémité proximale du tuyau, ou des tuyaux, d'insertion pour sortir le filtre par l'extrémité distale du tuyau, ou des tuyaux, d'insertion.

7. Filtre de thrombose selon la revendication 6, dans lequel lesdits premier et second modules (I, II) sont reliés par un élément élastique (1) s'étendant le long dudit axe longitudinal, caractérisé en ce que ledit élément élastique exerce une force sur lesdits modules en provoquant un accrochage des extrémités libres des aiguillons (31, 32) sur la paroi interne du vaisseau sanguin et un maintien du filtre dans la position souhaitée.

8. Filtre de thrombose selon la revendication 7, caractérisé en outre en ce que chaque module (I, II) dudit filtre comprend un fil central (21, 22), une extrémité de chaque fil central est ancrée à l'un desdits modules et s'étend de son ancrage le long dudit élément élastique vers l'autre module, lesdits fils centraux se terminant en extrémités libres sans atteindre ledit autre module.

9. Procédé de fabrication d'un filtre de thrombose comportant des mécanismes de couplage aux deux extrémités, caractérisé par les étapes, dans lesquelles :
(a) on réalise un tuyau comportant des extrémités ouvertes ;
(b) on introduit une extrémité d'un mécanisme de couplage dans l'une des extrémités ouvertes dudit tuyau ;
(c) on introduit les extrémités d'une pluralité d'aiguillons divergeants vers l'extérieur dans l'autre extrémité ouverte dudit tuyau ;
(d) on introduit une extrémité d'un fil central dans ladite autre extrémité ouverte dudit tuyau ;
(e) on remplit tout l'espace restant dans le tuyau entre ses extrémités ouvertes avec une matière de liaison qui durcira pour fixer le mécanisme de couplage, la pluralité d'aiguillons et le fil central dans le tuyau.

10. Procédé selon la revendication 9, comprenant les étapes supplémentaires, dans lesquelles :
(f) on réalise un second tuyau comportant des extrémités ouvertes ;
(g) on introduit une extrémité d'un mécanisme de couplage dans l'une des extrémités ouvertes dudit second tuyau ;
(h) on introduit les extrémités d'une pluralité d'aiguillons divergeants vers l'extérieur dans l'autre extrémité ouverte dudit second tuyau ;
(i) on introduit une extrémité d'un fil central dans ladite autre extrémité ouverte dudit second tuyau ;
(j) on relie une extrémité de l'élément élastique à ladite autre extrémité dudit tuyau ;
(k) on relie l'autre extrémité dudit élément élastique à ladite autre extrémité dudit second tuyau ; et
(l) on remplit tout l'espace restant dans ledit second tuyau entre ses extrémités ouvertes d'une matière de liaison qui durcira pour fixer le mécanisme de couplage, la pluralité d'aiguillons et le fil central dans le tuyau.

11. Procédé selon la revendication 9, dans lequel les aiguillons divergeants comportent des extrémités libres s'étendant dudit tuyau, lesquelles se trouvent le long de la surface d'un cône dont le sommet se trouve à l'intérieur dudit tuyau.

12. Procédé selon la revendication 10, dans lequel les aiguillons divergeants qui ont été liés dans ledit tuyau et dans ledit second tuyau comportent des extrémités libres s'étendant dudit tuyau et dudit second tuyau, lesquelles se trouvent le long des surfaces de cônes dont les sommets se trouvent à l'intérieur dudit tuyau et dudit second tuyau.

13. Procédé selon la revendication 12, dans lequel les aiguillons divergeants s'étendant dudit tuyau croisent les aiguillons divergeants s'étendant dudit second tuyau.
